# EUROPEAN PATENT SPECIFICATION

(11) **EP 2 011 500 B1**
(45) Date of publication and mention of the grant of the patent: **11.09.2013**
(21) Application number: 07741227.8
(22) Date of filing: 06.04.2007
(51) Int. Cl.: A61K 31/661, A23L 1/30, A23L 2/00, A61K 35/20, A61P 3/04, A23L 1/305, A23L 2/52

(54) **FAT ACCUMULATION INHIBITOR FOR THE TREATMENT OF METABOLIC SYNDROME**
FETTAKKUMULATIONSHEMMER ZUR BEHANDLUNG DES METABOLISCHEN SYNDROMS
INHIBITEUR DE L'ACCUMULATION DE GRAISSE POUR LE TRAITEMENT DU SYNDROME MÉTABOLIQUE

(30) Priority: 07.04.2006 JP 2006106164; 31.05.2006 JP 2006152507
(43) Date of publication of application: 07.01.2009
(73) Proprietor: MEGMILK SNOW BRAND Co., Ltd., Higashi-ku Sapporo (JP)
(72) Inventor: TANAKA, Reo, 1-1-2, Minamidai, Kawagoe-shi, Saitama 350-1165 (JP); ISOGAI, Tomoyuki, 1-1-2, Minamidai, Kawagoe-shi, Saitama 350-11 (JP); HARUTA, Yuko, 1-1-2, Minamidai, Kawagoe-shi, Saitama 350-11 (JP); MIURA, Susumu, 1-1-2, Minamidai, Kawagoe-shi, Saitama 350-11 (JP); KATO, Ken, 1-1-2, Minamidai, Kawagoe-shi, Saitama 350-11 (JP); YOSHIOKA, Toshimitsu, 1-1-2, Minamidai, Kawagoe-shi, Saitama 350-11 (JP); KAWAKAMI, Hiroshi, 1-1-2, Minamidai, Kawagoe-shi, Saitama 350-11 (JP); HIGURASHI, Satoshi, 1-1-2, Minamidai, Kawagoe-shi, Saitama 350-11 (JP); MATSUYAMA, Hiroaki, 1-1-2, Minamidai, Kawagoe-shi, Saitama 350-11 (JP)
(74) Representative: Grünecker, Kinkeldey, Stockmair & Schwanhäusser
(86) International application number: PCT/JP2007/057791
(87) International publication number: WO 2007/116981

(56) References cited:
- EP-A1- 1 424 074
- WO-A1-2006/006286
- JP-A- 01 279 837
- SPITSBERG V L: "Invited review: Bovine milk fat globule membrane as a potential nutraceutical", JOURNAL OF DAIRY SCIENCE, AMERICAN DAIRY SCIENCE ASSOCIATION, US, vol. 88, no. 7, 1 July 2005 (2005-07-01), pages 2289-2294, XP002436135, ISSN: 0022-0302, DOI: 10.3168/JDS.S0022-0302(05)72906-4
- SACHDEVA S ET AL: "RECOVERY OF PHOSPHOLIPIDS FROM BUTTERMILK USING MEMBRANE PROCESSING", KIELER WIRTSCHAFTLICHE FORSCHUNGSBERICHTE, VERLAG TH. MANN, GELSENKIRCHEN, DE, vol. 49, no. 1, 1 January 1997 (1997-01-01), pages 47-68, XP001014232, ISSN: 0023-1347
- YAMAUCHI K. ET AL.: 'Milk Sogo Jiten' KABUSHIKI KAISHA ASAKURA SHOTEN 1998, pages 378 - 380, XP003018179
- UENOGAWA S.: 'Series <Shokuhin no Kagaku> Chichi no Kagaku' KABUSHIKI KAISHA ASAKURA SHOTEN 1998, pages 19-27 - 185-191, XP003018180
- CORREDIG M. ET AL.: 'Production of a Novel Ingredient from Buttermilk' J.DAIRY SCI vol. 86, 1998, pages 2744 - 2750, XP003018181

## Description

### TECHNICAL FIELD

The present invention relates to an agent for use in the prevention and/or treatment of metabolic syndrome which comprises a sphingosine-containing phospholipid, particularly sphingomyelin, as an active ingredient, and a food or drink for use in the prevention and/or treatment of metabolic syndrome which comprises a sphingosine-containing phospholipid.

### BACKGROUND OF THE INVENTION

In recent years, increase of a risk of causing lifestyle-related diseases, such as obesity, hypertension, hyperlipemia, diabetes mellitus and the like due to disorder of dietary life, chronic lack of exercise, too much stress and the like, became a subject of the discussion. Advance of the symptoms of these lifestyle-related diseases results in more serious diseases such as arteriosclerosis, myocardial infarction and the like. Regarding the disorder of dietary life among the causes of lifestyle-related diseases, its large element is that dietary life of Japanese people changed to meats as the core like the case of Europeans and Americans, and therefore it became markedly high calories in comparison with conventional Japanese meal.

As a prevention of the lifestyle-related diseases caused by such a high calorie meal, roughly two methods can be considered. One is to lower concentration of a bad cholesterol and neutral fat (triglyceride) in blood, that is, to improve a lipid metabolism, and the other is to prevent obesity by inhibiting fat accumulation itself. In general, both are apt to be regarded as the same but their mechanisms are different in reality, and a medicine which improves the lipid mechanism but does not inhibit weight gain (Patent Reference 1) and a medicine which inhibits weight gain but is not concerned in the improvement of the lipid mechanism (Patent Reference 2) are respectively present.

Regarding the latter fat accumulation inhibition, the prevention and dissolution of obesity have been highlighted, not only for reasons of health but also from the cosmetic point of view. As its treatment, a drug therapy, an exercise therapy, a diet restriction and the like have been attempted. However, it is the present situation that an effect can be expected from the drug therapy, but on the contrary, it causes a necessity to take side-effects into consideration, and that the exercise therapy and diet restriction being generally carried out accompany a temporal or mental difficulty in continuously carrying out, and the success ratio is low. In addition, it cannot be ignored also that too much restriction of diet involves a risk of leading to nutritional disorders and anorexia. Under such circumstances, a fat accumulation inhibitor or a food or drink having an action of inhibition of fat accumulation, which can be ingested simply and safely in the daily dietary life, has been desired.

A phospholipid is a species of a daily ingested lipid, and it has been reported that a soybean- or egg yolk-derived phospholipid has the action of inhibition of fat accumulation (Patent Reference 3). However, a composition of the phospholipid greatly varies depending on its derivation, and regarding a milk-derived phospholipid, it has been reported only that it has an action of improvement of lipid metabolism (Patent Reference 4). That is, nothing is known about the action of inhibition of fat accumulation of the milk-derived phospholipid. In this connection, the Patent Reference 4 discloses an action of inhibition of neutral fat accumulation in a liver of the milk-derived phospholipid, but with regard to the fat accumulation in the liver, the liver cannot sufficiently treat the same because of the increase of neutral fat concentration in blood, and as a result, the surplus fat is accumulated directly. On the other hand, the one disclosed by the invention is an action of inhibition of incorporation of a lipid into a fat cell and is completely different from the above-mentioned action.

In addition, in recent years, the number of people showing morbid sates of diabetes mellitus, hypertension, hyperlipemia and arteriosclerosis as lifestyle-related diseases has been increasing accompanied by the Europeanized and Americanized life stile. Particularly, the death by cardiovascular diseases and cerebrovascular diseases occupies about one third of the cause of death, and the number thereof is increasing every year, so that a countermeasure for this is becoming a national problem. The degree of the risk of onset of these arteriosclerotic diseases is considerably increased by the accumulation of hypertension, hyperlipemia, impaired glucose tolerance and the like risk factors. This state of accumulating the risk factors is called a metabolic syndrome and has been broadly recognized.

According to an investigation on 120,000 enterprise workers in Japan, it is said that the risk of the onset of heart diseases becomes 5 times for a parson who has one risk factor among "obesity", "hypertension", "hyperglycemia", "hypertriglyceride(neutral fat)emia" and "hypercholesterolemia" even when it is a mild case, and that becomes 10 times for a parson who has two of them and 31 times for a parson who jointly has 3 or 4 of them. In addition, according to an investigation by the Ministry of Health, Labor and Welfare, it is reported that the number of hypertensive patients is 39,000,000, that of hyperlipemia is 22, 000, 000, that of diabetes mellitus (including reserve patients) is 16,200,000 and that of obesity is 4,680,000, and these patients are increasing every year.

The metabolic syndrome is "a multiple risk factor syndrome in which accumulation of visceral fat complicates with two or more of insulin resistance, glucose metabolism disorder, dislipidemia and hypertension, based on the former, which is a morbid state of easily causing arteriosclerosis", and the accumulation of visceral fat is certainly its basic factor. A fat tissue as a largest secretory tissue in a living body is concerned in the maintenance of homeostasis in the living body by producing various endocrine factors. However, it has been found that excess accumulation of visceral fat leads a loss of secretory balance of the endocrine factors to induce various morbid states. Particularly, the secretion quantity of a plasminogen activator inhibitor (PAI-1), a tumor necrosis factor (TNF-α), leptin and the like endocrine factors increases accompanied by the accumulation of visceral fat to induce thrombosis, insulin resistance, glucose metabolism disorder, hypertension and the like.

On the other hand, it is known that an adiponectin which is specifically secreted by the fat tissue is generally present in blood at a high concentration, but the concentration thereof decreases accompanied by the accumulation of visceral fat. Since it is known that the adiponectin has anti-diabetes, anti-arteriosclerosis, anti-inflammatory action, anti-hypertension and the like various physiological functions, acceleration of the increase of the adiponectin concentration or inhibition of the decrease of the adiponectin concentration in blood is very important for the prevention and treatment of the metabolic syndrome.

Conventionally, a drug therapy has also been carried out as a countermeasure for individual morbid state of the metabolic syndrome, but the necessity for a prescription, involvement of side effects and the like are coming into a subject of discussion. In addition, since it has been found that even when a treatment is carried out for one morbid state, it develops into a serious morbid state triggered by other morbid state, it becomes necessary to adjust secretory valance of fat cell-derived endocrine factors which are present in the upstream of these states. Because of this, it is considered that reexamination of the exercise therapy, diet restriction and the like daily life is important rather than the drug therapy for the prevention and treatment of the metabolic syndrome caused by the accumulation of the visceral fat. Accordingly, a food or drink which can be daily ingested, has high safety even when ingested over a prolonged period of time and is effective for the prevention and treatment of the metabolic syndrome caused by the accumulation of the visceral fat, has been desired.

CA 2 573 261 and EP 1 424 074 disclose compositions useful in the prevention of obesity, which comprise milk phosholipids.
Patent Reference 1: JP-A-2002-326946
Patent Reference 2: JP-A-2004-99539
Patent Reference 3: JP-A-10-84880
Patent Reference 4: JP-A-2001-275614

### PROBLEMS OF THE INVENTION

The objective of the invention is to provide an agent and a food or drink for use in the prevention and/or treatment of metabolic syndrome.

### MEANS FOR SOLVING THE PROBLEMS

The problems are solved by an agent for use in the prevention and/or treatment of metabolic syndrome, which comprises a sphingosine-containing phospholipid as an active ingredient, and a food or drink for use in the prevention and/or treatment of metabolic syndrome, which comprises the sphingosine-containing phospholipid.

The inventors have conducted intensive studies on the search of a component which lowers the visceral fat considered to be a cause of the metabolic syndrome and a component which does not lower the adiponectin concentration considered to increase the risk of cardiovascular diseases when its concentration in blood is lowered, among milk components. As a result, a markedly high action of inhibition of visceral fat accumulation and action of acceleration of increase and/or inhibition of decrease of an adiponectin concentration in blood were found in a sphingosine-containing phospholipid, thereby resulting in the accomplishment of the invention.

Preferred embodiments of the present invention are set forth in the dependent claims.

### ADVANTAGE OF THE INVENTION

The agent and food or drink for use according to the invention are used for the prevention and treatment of metabolic syndrome which is considered to be caused by accumulating the visceral fat or lowering the adiponectin in blood. In addition, since the agent and food or drink for use according to the present invention, comprise a sphingosine-containing phospholipid, particularly sphingomyelin, they can be supplied in a large amount with relatively low cost and their safety is markedly high.

### BEST MODE FOR CARRYING OUT THE INVENTION

The invention provides an agent for use in the prevention and/or treatment of metabolic syndrome, which comprises a sphingosine-containing phospholipid as an active ingredient, and a food or drink for the same use. As the sphingosine-containing phospholipid, sphingomyelin is particularly desirable. This is because the sphingomyelin has a markedly high action of inhibition of the visceral fat accumulation and action of acceleration of increase and/or inhibition of decrease of an adiponectin concentration in blood.

Though the sphingomyelin is contained in milk in a large amount of from 20 to 30% bymass inphospholipid, studies on its functions are limited to a cell level, and the finding on its physiological functions in a living body is little. Thus, its effectiveness as a component of nutrient has not so far been recognized.

Regarding an application of the sphingomyelin, an anti-inflammatory external agent, an agent for improving function of digestion and absorption of lipid, an agent for treating dysfunctional disease of intestinal movement (JP-A-5-186330, JP-A-11-269074, JP-A-2003-252-765) and the like are known, but the action of inhibition of visceral fat accumulation and the action of acceleration of increase and/or inhibition of decrease of an adiponectin concentration in blood have not been revealed.

The sphingosine-containing phospholipid, particularly sphingomyelin, used in the invention may be purified or used as a sphingomyelin-containing phospholipid. Though the sphingomyelin is frequently contained in an animal brain and milk fat, it is desirably a milk-derived phospholipid from the viewpoint of carrying out the invention.

As a material of the milk-derived sphingomyelin, for example, a raw milk, a whey protein concentrate (WPC), a butter curd, and a butter serum can be exemplified. As a method for preparing the milk-derived sphingomyelin, for example, a method for extracting with ether or acetone for obtaining a sphingomyelin-containing phospholipid fraction from a raw milk, or WPC (JP-A-3-47192), a method which uses a water-soluble fraction containing a butter curd or a butter serum, and conventionally known method can be exemplified. The sphingomyelin content of the fraction obtained by employing these materials and methods is about 28% by mass and about 9% by mass, respectively.

In addition, sphingomyelin having improved purity can be obtained by purifying the aforementioned sphingomyelin-containing phospholipid fraction through dialysis, ammonium sulfate fractionation, gel filtration, an isoelectric precipitation, ion exchange chromatography, solvent fractionation, ultrafiltration (UF), and microfiltration (MF) techniques.

The sphingomyelin or sphingomyelin-containing phospholipid obtained by the above-mentioned method can be made into liquid, powder, or tablets and can be directly administered orally. In addition, a phospholipid composition containing not only the sphingomyelin but also an effective amount of phosphatidylcholine defined as a necessary amount of human nutrition may be used.

As the dosage forms of the agent for use according to the present invention, a tablet, a capsule, a granule, powdered materials, and a powder can be exemplified.

In addition, as a food or drink in which comprises a sphingosine-containing phospholipid , particularly sphingomyelin, for use according to the invention, a milk, a milk drink, a coffee drink, a juice, a jelly, a biscuit, a bread, a noodle, a sausage, and various types of powdered milk, as well as a nutritious composition aimed at a suckling, a baby, or a low birth weight infant can be exemplified. Since these can be daily ingested and have the action of inhibition of visceral fat accumulation and action of acceleration of increase and/or inhibition of decrease of an adiponectin concentration in blood, they are useful in preventing and treating the metabolic syndrome caused by the increase of visceral fat and decrease of the adiponectin in blood.

In order to exert the action of inhibition of visceral fat accumulation and action of acceleration of increase and/or inhibition of decrease of an adiponectin concentration in blood by the agent and food or drink for use according to the present invention, the blending amounts may be adjusted such a manner that from 0.1 to 5,000 mg per day of a sphingosine-containing phospholipid, particularly sphingomyelin, can be ingested in the case of adult.

The following describes the invention further in detail with reference to examples and test examples.

### EXAMPLES

### [Example 1]

A protease was allowed to react with 10% by mass aqueous solution of a whey protein concentrate (WPC), and the thus obtained reaction liquid was extracted with chloroform-methanol (2:1) and then concentrated, and further extracted with acetone to obtain a complex lipid fraction. Next, this complex lipid fraction was treated with a fluorosilyl column chromatography and subjected to a stepwise extraction with chloroform-methanol solution to obtain a phospholipid fraction. This phospholipid fraction was treated with a silica-gel chromatography and subjected to a stepwise extraction with chloroform-methanol solution, and the product was freeze-dried to obtain sphingomyelin. When this sphingomyelin was treated with a thin-layer chromatography and then subjected to a development of color with Dittmer' s reagent and measured by a densitometry method, the sphingomyelin content was 95.2% by mass. The sphingomyelin obtained in this manner inhibits visceral fat accumulation and accelerates the increase and/or inhibits the decrease of an adiponectin concentration in blood.

### [Test Example 1]

### (Verification of action of acceleration of increase and/or inhibition of decrease of adiponectin concentration in blood)

Using the sphingomyelin obtained in Example 1, an action of acceleration of increase and/or inhibition of decrease of an adiponectin concentration in blood was verified. An animal test was carried out using 8 animals per group by a group in which a high fat feed blended with sphingomyelin was fed (sphingomyelin diet group) and a group in which a high fat feed not blended with sphingomyelin was fed (high fat diet group). The high fat diet was prepared using a milk casein as a protein source and a butter oil as a lipid source. In the animal test, a feed was provided until the 4^{th} week after rearing, and thereafter the group was divided into 5 groups, and the high fat feed or sphingomyelin-blended high fat feed was provided until the 8^{th} week. Blood collection was carried out on the 4^{th} week and on the 8^{th} week, and an adiponectin concentration in blood was measured using a Mouse/Rat Adiponectin ELISA Kit (manufactured by Otsuka Pharmaceutical Co., Ltd.).

The results are shown in Table 1. According to this, it was revealed that the adiponectin concentration in blood in the high fat diet group decreases with the lapse of time, while it increases in the sphingomyelin diet group. That is, it was found that increase of the adiponectin concentration in blood is accelerated or its decrease is inhibited by the ingestion of sphingomyelin.

**[Table 1]**

| Feed group | Adiponectin concentration in blood (µg/ml) | |
|---|---|---|
| | 4 weeks | 8 weeks |
| High fat diet | 11.54 | 11.07 |
| Sphingomyelin (Example 7) diet | 10.88 | 15.65 |

### [Test Example 2]

### (Verification of action of visceral fat accumulation inhibition)

Using the sphingomyelin obtained in Example 1, an action of inhibition of visceral fat accumulation was verified. An animal test was carried out using 8 animals per group by a group in which a high fat feed blended with sphingomyelin was fed (sphingomyelin diet group) and a group in which a high fat feed not blended with sphingomyelin was fed (high fat diet group). In the animal test, the high fat feed was provided until the 4^{th} week after rearing, and thereafter the group was divided into 5 groups, and the high fat feed or sphingomyelin-blended high fat feed was provided until the 8^{th} week. By carrying out dissection on the 8^{th} week, the amount of visceral fats (mesenteric, peri-testicular, perirenal, posterior abdominal) was measured.

The results are shown in Table 2. According to this, it was revealed that, in comparison with the amount of the visceral fat of the high fat diet group, the sphingomyelin diet group generally shows low values. That is, it was found that the accumulation of the visceral fat is inhibited by ingesting sphingomyelin.

**[Table 2]**

| Feed group | Amount of visceral fat (g) | | | |
|---|---|---|---|---|
| | Mesenteric | peritesticular | Perirenal | posterior abdominal |
| High fat diet | 2.96 | 3.68 | 0.98 | 2.56 |
| Sphingomyelin (Example 7) diet | 2.19 | 3.22 | 0.74 | 2.11 |

### [Example 2]

Materials were mixed based on the mixture shown in Table 3, and that was granulated and then packed in a capsule to produce capsules which inhibit visceral fat accumulation or accelerate the increase and/or inhibit the decrease of an adiponectin concentration in blood.

**[Table 3]**

| | (% by mass) |
|---|---|
| Sphingomyelin (Example 7) | 20 |
| Lactose | 24.5 |
| Soluble starch | 55 |
| Magnesium stearate | 0.5 |

### [Example 3]

Materials were mixed based on the mixture shown in Table 4 and that was packed in a container and then heat-sterilized to produce a drink which inhibits visceral fat accumulation or accelerates the increase and/or inhibits the decrease of an adiponectin concentration in blood.

**[Table 4]**

| | (% by mass) |
|---|---|
| Sphingomyelin (Example 7) | 2.5 |
| Sugar | 7.5 |
| Citric acid | 0.6 |
| Apple juice | 10 |
| Water | 79.4 |

## Claims

1. An agent for use in the prevention and/or treatment of metabolic syndrome which comprises a sphingosine-containing phospholipid as an active ingredient.

2. The agent of claim 1 for use according to claim 1, wherein the sphingosine-containing phospholipid is sphingomyelin.

3. A food or drink for use in the prevention and/or treatment of metabolic syndrome which comprises a sphingosine-containing phospholipid.

4. The food or drink of claim 3 for use according to claim 3, wherein the sphingosine-containing phospholipid is sphingomyelin.

5. Use of a sphingosine-containing phospholipid in the preparation of a medicament for the prevention and/or treatment of metabolic syndrome.

6. The use of claim 5, wherein the sphingosine-containing phospholipid is sphingomyelin.

7. Use of a sphingosine-containing phospholipid in the preparation of a food or drink for the prevention and/or treatment of metabolic syndrome.

8. The use of claim 7, wherein the sphingosine-containing phospholipid is sphingomyelin.

9. The agent of claim 1 or 2 for use according to claim 1 or 2 or the food or drink of claim 3 or 4 for use according to claim 3 or 4, wherein the sphingosine-containing phospholipid is a milk-derived sphingosine-containing phospholipid.

10. The use according to claims 5 to 8, wherein the sphingosine-containing phospholipid is a milk-derived sphingosine-containing phospholipid.

## Patentansprüche

1. Mittel zur Verwendung bei der Verhütung und/oder Behandlung des metabolischen Syndroms, welches ein sphingosinhaltiges Phospholipid als einen Wirkstoff umfasst.

2. Mittel nach Anspruch 1 zur Verwendung gemäß Anspruch 1, wobei das sphingosinhaltige Phospholipid Sphingomyelin ist.

3. Nahrungsmittel oder Getränk zur Verwendung bei der Verhütung und/oder Behandlung des metabolischen Syndroms, welches ein sphingosinhaltiges Phospholipid umfasst.

4. Nahrungsmittel oder Getränk nach Anspruch 3 zur Verwendung gemäß Anspruch 3, wobei das sphingosinhaltige Phospholipid Sphingomyelin ist.

5. Verwendung eines sphingosinhaltigen Phospholipids bei der Herstellung eines Medikaments für die Verhütung und/oder Behandlung des metabolischen Syndroms.

6. Verwendung nach Anspruch 5, wobei das sphingosinhaltige Phospholipid Sphingomyelin ist.

7. Verwendung eines sphingosinhaltigen Phospholipids bei der Herstellung eines Nahrungsmittels oder Getränks für die Verhütung und/oder Behandlung des metabolischen Syndroms.

8. Verwendung nach Anspruch 7, wobei das sphingosinhaltige Phospholipid Sphingomyelin ist.

9. Mittel nach Anspruch 1 oder 2 zur Verwendung gemäß Anspruch 1 oder 2, oder Nahrungsmittel oder Getränk nach Anspruch 3 oder 4 zur Verwendung gemäß Anspruch 3 oder 4, wobei das sphingosinhaltige Phospholipid ein aus Milch gewonnenes sphingosinhaltiges Phospholipid ist.

10. Verwendung gemäß den Ansprüchen 5 bis 8, wobei das sphingosinhaltige Phospholipid ein aus Milch gewonnenes sphingosinhaltiges Phospholipid ist.

## Revendications

1. Agent destiné à être utilisé dans la prévention et/ou le traitement d'un syndrome métabolique, qui comprend un phospholipide contenant de la sphingosine en tant qu'ingrédient actif.

2. Agent selon la revendication 1 destiné à être utilisé selon la revendication 1, dans lequel le phospholipide contenant de la sphingosine est la sphingomyéline.

3. Aliment ou boisson destiné(e) à être utilisé(e) dans la prévention et/ou le traitement d'un syndrome métabolique, qui comprend un phospholipide contenant de la sphingosine.

4. Aliment ou boisson selon la revendication 3 destiné(e) à être utilisé(e) selon la revendication 3, dans lequel/laquelle le phospholipide contenant de la sphingosine est la sphingomyéline.

5. Utilisation d'un phospholipide contenant de la sphingosine dans la préparation d'un médicament destiné à la prévention et/ou au traitement d'un syndrome métabolique.

6. Utilisation selon la revendication 5, dans laquelle le phospholipide contenant de la sphingosine est la sphingomyéline.

7. Utilisation d'un phospholipide contenant de la sphingosine dans la préparation d'un aliment ou d'une boisson destiné(e) à la prévention et/ou au traitement d'un syndrome métabolique.

8. Utilisation selon la revendication 7, dans laquelle le phospholipide contenant de la sphingosine est la sphingomyéline.

9. Agent selon la revendication 1 ou 2 destiné à être utilisé selon la revendication 1 ou 2, ou aliment ou boisson selon la revendication 3 ou 4 destiné(e) à être utilisé (e) selon la revendication 3 ou 4, dans lequel/laquelle le phospholipide contenant de la sphingosine est un phospholipide contenant de la sphingosine dérivé du lait.

10. Utilisation selon les revendications 5 à 8, dans laquelle le phospholipide contenant de la sphingosine est un phospholipide contenant de la sphingosine dérivé du lait.
